# EUROPÄISCHE PATENTSCHRIFT

(11) **EP 0 952 150 B1**
(45) Veröffentlichungstag und Bekanntmachung des Hinweises auf die Patenterteilung: **02.11.2006**
(21) Anmeldenummer: 99105784.5
(22) Anmeldetag: 22.03.1999
(51) Int. Cl.: C07D 213/64

(54) **Verfahren zur Herstellung von Phenoxypropinsäurepropargylestern**
Process for the production of phenoxypropionic acid propargyl esters
Procédé de préparation d'esters propargyliques de l'acide phénoxy propionique

(30) Priorität: 24.03.1998 CH 68798
(43) Veröffentlichungstag der Anmeldung: 27.10.1999
(73) Patentinhaber: Syngenta Participations AG, 4058 Basel (CH)
(72) Erfinder: Seifert, Gottfried, 4312 Magden (CH); Sting, Andrea Rolf, 7503 Samedan (CH); Urwyler, Bernhard, 4106 Therwil (CH)
(74) Vertreter: Arunasalam, Velautha-Cumaran

(56) Entgegenhaltungen:
- EP-A- 0 248 968
- E. C. BOLTON ET AL.: "Chiral Diacetylenes derived from L-alpha-Amino Acids" J. CHEM. RES. MINIPRINT, Bd. 7, 1992, Seiten 1601-1615, XP002113234
- M. GAUDEMAR: "Réarrangement propargyl-allénylique dans le substitutions hétérolytiques et dans les réactions de transfert électronique" ANN. CHIM. (PARIS), Bd. 13, Nr. 1, 1956, Seiten 161-213, XP002113235
- M. G. VORONKOV ET AL.: "Propargyloxy derivatives of organic and organometallic compounds" J. APP. CHEM. USSR, Bd. 65, Nr. 12.2, 1992, Seiten 2310-2314, XP002113236
- L. J. LOEFFLER ET AL.: "Antineoplastic Agents. 2. Structure-Activity Studies on N-Protected Vinyl, 1,2-Dibromoethyl, and Cyanomethyl Esters of Several Amino Acids" J. MED. CHEM., Bd. 20, Nr. 12, 1977, Seiten 1584-1588, XP002113237
- A. LOUPY ET AL.: "Easy and efficient S(N)Ar reactions on halopyridines in solvent free conditions" HETEROCYCLES, Bd. 32, Nr. 10, 1991, Seiten 1947-1953, XP002113238

## Beschreibung

Die vorliegende Erfindung betrifft ein Verfahren zur Herstellung von (R)(+)-2-[4-(5-Chlor-3-fluorpyridin-2-yloxy)-phenoxy]-propionsäurepropinylester.

(R)(+)-2-[4-(5-Chlor-3-fluorpyridin-2-yloxy)-phenoxy]-propionsäurepropinylester besitzt herbizide Wirkung und ist beispielsweise in EP-A-0 248 968 beschrieben. 4-(5-Chlor-3-fluorpyridin-2-yloxy)-phenoxy-propionsäurederivate können beispielsweise gemäß EP-A-0 439 857 hergestellt werden, indem man 5-Chlor-2,3-difluorpyridin mit entsprechenden 4-Hydroxypropionsäureestern in Gegenwart einer wasserfreien Base und in Abwesenheit eines Lösungsmittels umsetzt. Dieses Verfahren ist jedoch zur Herstellung von (R)-2-[4-(5-Chlor-3-fluorpyridin-2-yloxy)-phenoxy]-propionsäurepropinylester ungeeignet, da die Dreifachbindung des Hydroxypropionsäureesters unter Prozeßbedingungen und unter basischen Bedingungen zur Bildung von Polymeren neigt. Ferner ist dieses Verfahren besonders unter dem Aspekt der Sicherheit problematisch, da die Reaktionsmischung wegen des hohen thermischen Potentials dieser Dreifachbindung nur unter Risiko ohne Lösungsmittel erhitzt werden kann.

Gemäß EP-A-0 248 968, Seiten 12 bis 14, erhält man (R)(+)-2-[4-(5-Chlor-3-fluorpyridin-2-yloxy)-phenoxy]-propionsäurepropinylester, indem man
a) in einem ersten Schritt eine Verbindung der Formel A 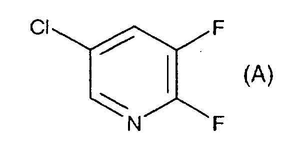 in Dimethylsulfoxid mit einem Gemisch aus Hydrochinon und Kaliumhydroxid in Dimethylsulfoxid zu einer Verbindung der Formel B 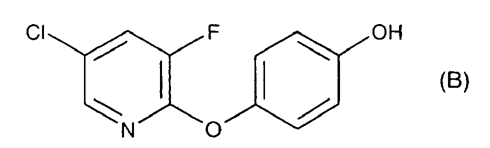 umsetzt, diese
b) in einem zweiten Schritt in Dimethylsulfoxid in Gegenwart von Kaliumcarbonat mit S(-)-Milchsäuremethylester-tosylat zur Verbindung der Formel C 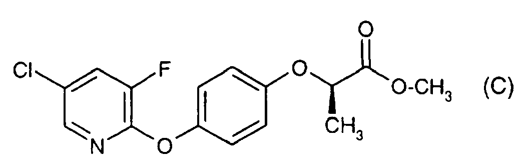 umsetzt, diese
c) in einem dritten Schritt in Dioxan in Gegenwart von Natronlauge zur Verbindung der Formel D 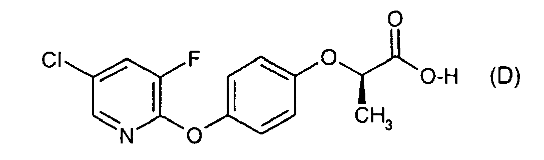 umsetzt, diese
d) in einem vierten Schritt in Toluol mit Thionylchlorid zur Verbindung der Formel (E) 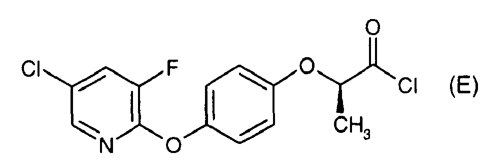 umsetzt, die schließlich ohne weitere Isolierung
e) mit einem Gemisch aus Triethylamin und Propinol in Toluol zum (R)(+)-2-[4-(5-Chlor-3-fluorpyridin-2-yloxy)-phenoxy]-propionsäurepropinylester umgesetzt wird.

Dieses Verfahren besitzt den schwerwiegenden Nachteil, daß durch die vierstufige Reaktionsführung aufwendige Abtrennungs- und Reinigungsschritte erforderlich sind. Dies führt zu erheblichen Ausbeuteverlusten. Ferner muß das Lösungsmittel während der Durchführung des Verfahrens zweimal gewechselt werden. Dies erfordert zusätzliche zeitraubende und kostenintensive Destillationsschritte. Daher ist das bekannte Verfahren insbesondere für die großtechnische Anwendung nicht optimal.

Es ist daher die Aufgabe der vorliegenden Erfindung ein Verfahren bereitzustellen, daß die Herstellung von (R)(+)-2-[4-(5-Chlor-3-fluorpyridin-2-yloxy)-phenoxy]-propionsäurepropinylester auf einfacherer Weise, in höherer Reinheit und in höheren Ausbeuten ermöglicht.

Es wurde nun gefunden, daß man (R)(+)-2-[4-(5-Chlor-3-fluorpyridin-2-yloxy)-phenoxy]-propionsäurepropinylester der Formel I 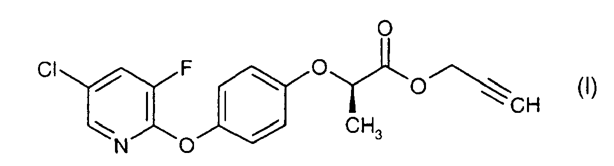 besonders vorteilhaft herstellen kann, indem man eine Verbindung der Formel II 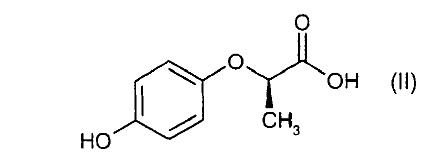 in einem inerten organischem Lösungsmittel ohne Isolierung von Zwischenprodukten mit M₂CO₃, worin M für Natrium oder Kalium steht, in die Verbindung der Formel III 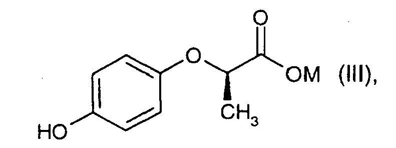 worin M für Natrium oder Kalium steht, überführt, diese mit der Verbindung der Formel IV 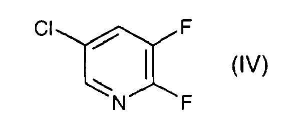 zur Verbindung der Formel V 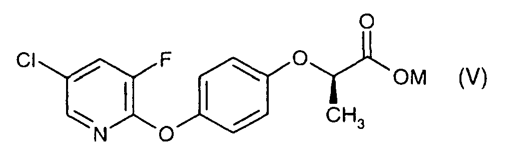 worin M für Natrium oder Kalium steht, umsetzt und diese mit einer Verbindung der Formel VI 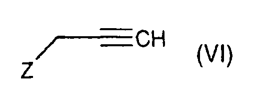 worin Z eine Abgangsgruppe wie Phenylsulfonyl, Tosyl, Methylsulfonyl, Nosyl, Bromphenyl, Cl-, Br- oder CICO- bedeutet, in die Verbindung der Formel I überführt.

Die Ausgangsverbindungen können in stöchiometrischen Mengen eingesetzt werden. Vorzugsweise wird die Verbindung der Formel IV in einem Überschuß von 0,05 bis 0,3 Äquivalenten, besonders bevorzugt von 0,1 Äquivalenten in Bezug auf die Verbindung der Formel III eingesetzt. Die Verbindung der Formel VI wird vorzugsweise mit einem Überschuß von 0,05 bis 0,15 Äquivalenten eingesetzt.

Im Rahmen der vorliegenden Erfindung steht M in M₂CO₃ vorzugsweise für Kalium.

Als inertes organisches Lösungsmittel eignen sich im Rahmen der vorliegenden Erfindung insbesondere Ketone, Ester und Ether, vorzugsweise eignen sich als Lösungsmittel Dimethylformamid, Dimethylsulfoxid, N-Methylpyrolidon oder Acetonitril. Besonders bevorzugt sind Dimethylformamid und Acetonitril, ganz besonders bevorzugt Dimethylformamid. In einer bevorzugten Ausführungsform des erfindungsgemäßen Verfahrens steht Formel VI Z für Chlor. Das erfindungsgemäße Verfahren kann bei erhöhten Temperaturen, insbesondere bei 40 bis 120 °C durchgeführt werden. Bevorzugt ist ein Temperaturbereich von 60 bis 90 °C, ganz besonders bevorzugt von 70 bis 75°C.

Die Umsetzung der Formel III mit der Formel IV kann zur Beschleunigung der Reaktion in Gegenwart eines Phasentransferkatalysators durchgeführt werden. Geeignete Phasentransferkatalysatoren sind beispielsweise quartäre Ammoniumsalze, quartäre Phosphoniumsalze oder Kronenether.

Die Ausgangsverbindungen der Formeln II, IV und VI sind bekannt oder lassen sich nach bekannten Verfahren herstellen. Die Verbindung der Formel IV ist beispielsweise in EP-A-0 248 968 , die Verbindung der Formel II in EP-A-0 083 556 beschrieben. Verbindungen der Formel VI, worin Z für Chlor steht, können z.B. gemäß J. Am. Chem. Soc. 77, 1831 (1955) hergestellt werden, wobei für diese Reaktion Pyridin und vorzugsweise 5-Ethyl-2-methylpyridin geeignete Basen sind.

Das erfindungsgemäße Verfahren unterscheidet sich von den bekannten Verfahren insbesondere dadurch, daß es als Eintopfverfahren ohne Lösungsmittelwechsel durchgeführt werden kann. Dadurch ist nicht nur der apparative Aufwand wesentlich geringer, sondern es wird auch durch die Vermeidung von aufwendigen Abtrenn- und Destillationsschritten ein erheblicher Zeitgewinn erzielt. Ferner ist die durch das erfindungsgemäße Verfahren ermöglichte wesentliche Verringerung von Lösungsmittelabfällen ökologisch besonders vorteilhaft. Die geringere thermische Belastung des Produkts vermindert die Bildung von unerwünschten Nebenprodukten und der besonders selektive Reaktionsverlauf ermöglicht eine exaktere Dosierung der Reaktanden was wiederum zu höherer Ausbeute und einem in der Reinheit erheblich verbessertem Produkt.

### Herstellungsbeispiele:

### Beispiel H1: Herstellung von (R)(+)-2-[4-(5-Chlor-3-fluorpyridin-2-yloxy)-phenoxyl-propionsäurepropinylester:

182 g (R)-2-(p-Hydroxyphenoxy)-propionsäure 100% (1 Mol) in 600 g DMF überführt man durch Zugabe von 69 g Kaliumcarbonatpulver (0.5 Mol) bei 70°C unter CO₂-Abspaltung in das entsprechende Kaliumsalz. Zu dieser Lösung gibt man 193 g Kaliumcarbonat Pulver (1.4 Mol) und anschließend bei einer Temperatur von 70-75°C innerhalb von 30 Minuten 165 g 5-Chlor-2,3-difluorpyridin (1.1 Mol) zu. Nach 4 Stunden wird die so erhaltene Verbindung der Formel V ohne Isolierung bei einer Temperatur von 70-75°C direkt durch Zudosierung von 86 g Propargylchlorid (1.15) Mol als 60-70 %-ige toluolische Lösung innerhalb von 2 Stunden vollständig zum (R)(+)-2-[4-(5-Chlor-3-fluorpyridin-2-yloxy)-phenoxy]-propionsäurepropinylester umgesetzt. Die Salze werden abfiltriert, mit 300 g DMF in Portionen gewaschen und das Filtrat am Rotationsverdampfer im Vakuum bei einer Temperatur von 120°C zur Schmelze eingedampft. Die Rohschmelze des (R)(+)-2-[4-(5-Chlor-3-fluorpyridin-2-yloxy)-phenoxy]-propionsäurepropinylesters wird bei einer Temperatur von 50°C mit 300 g Ethanol / Wasser 9:1 versetzt, bei einer Temperatur von 30 bis 35°C angeimpft und auf eine Temperatur von 0 bis 5°C gekühlt. Der Kristallbrei wird auf einen Saugfilter gegeben, mit 70 g Ethanol / Wasser 9:1 gewaschen und im Vakuum bei einer Temperatur von 30°C getrocknet. Man erhält 307 g Aktivsubstanz mit einem Gehalt von 97 % (GC) , entsprechend einer Ausbeute von 85 % der Theorie bezogen auf (R)-2-(p-Hydroxyphenoxy)-propionsäure.

### Beispiel H2: Herstellung von (R)(+)-2-[4-(5-Chlor-3-fluorpyridin-2-yloxy)-phenoxyl-propionsäurepropinylester:

182 g (R)-2-(p-Hydroxyphenoxy)-propionsäure 100% (1 Mol) in 1500 g Acetonitril überführt man durch Zugabe von 69 g Kaliumcarbonatpulver (0.5 Mol) bei 70°C unter CO₂₋Abspaltung in das entsprechende Kaliumsalz. Anschließend setzt man der Reaktionsmischung 193 g Kaliumcarbonat Pulver (1.4 Mol) sowie 2 g Tetrabutylammoniumbromid als Phasentransferkatalysator zu und dosiert ferner bei einer Temperatur von 70 bis 75°C innerhalb von 30 Minuten 165 g 5-Chlor-2,3-difluorpyridin (1.1 Mol) zu. Nach 8 Stunden wird die so erhaltene Verbindung der Formel V ohne Isolierung bei einer Temperatur von 70-75°C direkt durch Zudosierung von 154 g Propargylmesylat (1.15) Mol als 60-70 %-ige toluolische Lösung innerhalb von 2 Stunden vollständig zum (R)(+)-2-[4-(5-Chlor-3-fluorpyridin-2-yloxy)-phenoxy]-propionsäurepropinylester umgesetzt. Die Aufarbeitung erfolgt analog Beispiel H1. Man erhält 304 g (R)(+)-2-[4-(5-Chlor-3-fluorpyridin-2-yloxy)-phenoxy]-propionsäurepropinylester mit einem Gehalt von 98 % (GC), entsprechend einer Ausbeute von 85 % der Theorie bezogen auf (R)-2-(p-Hydroxyphenoxy)-propionsäure.

### Beispiel H3: Herstellung von (R)(+)-2-[4-(5-Chlor-3-fluorpyridin-2-yloxy)-phenoxyl-propionsäurepropinylester:

Wird das Propargylchlorid von Beispiel H1 durch 226 g (1.15 Mol) Benzosulfosäurepropargylester ersetzt, erhält man 305 g (R)(+)-2-[4-(5-Chlor-3-fluorpyridin-2-yloxy)-phenoxy]-propionsäurepropinylester mit einem Gehalt von 96 % (GC), entsprechend einer Ausbeute von 84 % der Theorie bezogen auf (R)-2-(p-Hydroxyphenoxy)-propionsäure.

### Beispiel H4: Herstellung von (R)(+)-2-[4-(5-Chlor-3-fluorpyridin-2-yloxy)-phenoxy]-propionsäurepropinylester:

182 g (R)-2-(p-Hydroxyphenoxy)-propionsäure 100% (1 Mol) in 1500 g Acetonitril überführt man durch Zugabe von 69 g Kaliumcarbonatpulver (0.5 Mol) bei 70°C unter CO₂₋Abspaltung in das entsprechende Kaliumsalz. Anschließend setzt man der Reaktionsmischung 193 g Kaliumcarbonatpulver (1.4 Mol) sowie 2 g Tetrabutylammoniumbromid als Phasentransferkatalysator zu und dosiert ferner bei einer Temperatur von 70 bis 75°C innerhalb von 30 Minuten 165 g 5-Chlor-2,3-difluorpyridin (1.1 Mol) zu. Nach 8 Stunden wird die so erhaltene Verbindung der Formel V ohne Isolierung bei einer Temperatur von 70-75°C direkt durch Zudosierung von Chlorameisensäurepropargylester, wobei CO₂ freigesetzt wird, vollständig zum (R)(+)-2-[4-(5-Chlor-3-fluorpyridin-2-yloxy)-phenoxy]-propionsäurepropinylester umgesetzt. Die Aufarbeitung erfolgt analog Beispiel H1. Man erhält (R)(+)-2-[4-(5-Chlor-3-fluorpyridin-2-yloxy)-phenoxy]-propionsäurepropinylester mit einem Gehalt von 97 % (GC) , entsprechend einer Ausbeute von 80 % der Theorie bezogen auf (R)-2-(p-Hydroxyphenoxy)-propionsäure.

## Patentansprüche

1. Verfahren zur Herstellung von (R)(+)-2-[4-(5-Chlor-3-fluorpyridin-2-yloxy)-phenoxy]-propionsäurepropinylester der Formel I 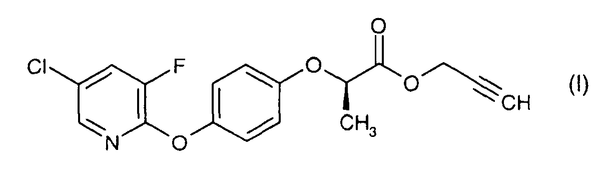 **dadurch gekennzeichnet, daß** man eine Verbindung der Formel II 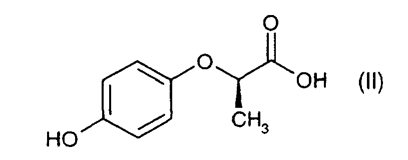 in einem inerten organischem Lösungsmittel ohne Isolierung von Zwischenprodukten mit M₂CO₃, worin M für Natrium oder Kalium steht, in die Verbindung der Formel III 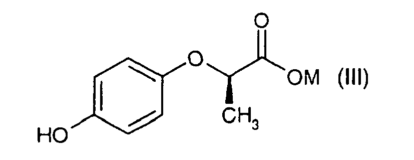 worin M für Natrium oder Kalium steht
überführt, diese mit der Verbindung der Formel IV 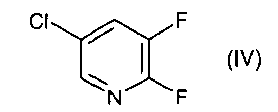 zur Verbindung der Formel V 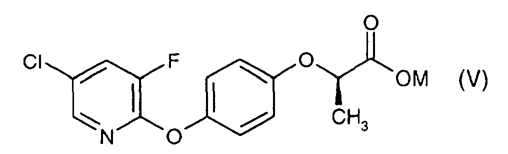 worin M für Natrium oder Kalium steht, umsetzt und diese mit einer Verbindung der Formel VI 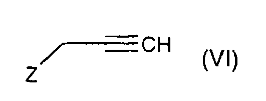 worin Z Phenylsulfonyl, Tosyl, Methylsulfonyl, Nosyl, Bromphenyl, Cl-, Br- oder CICObedeutet, in die Verbindung der Formel I überführt.

## Claims

1. Process for the preparation of (R)(+)-2-[4-(5-chloro-3-fluoropyridin-2-yloxy)-phenoxy]-propionic acid propinyl ester of formula I 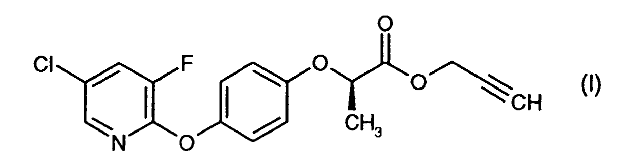 **characterised by** converting a compound of formula II 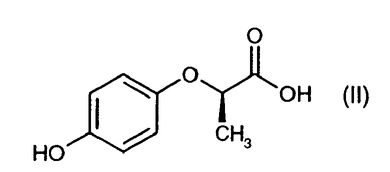 in an inert organic solvent, without isolation of the intermediate products, with M₂CO₃, in which M is sodium or potassium, into the compound of formula III 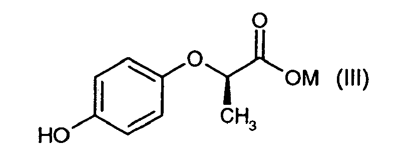 in which M is sodium or potassium,
reacting this with the compound of formula IV 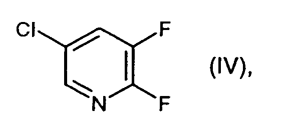 to form the compound of formula V 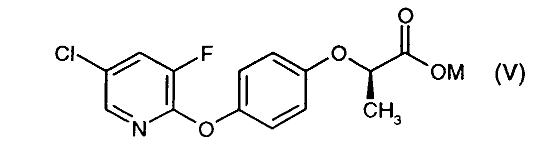 wherein M is sodium or potassium, and converting this compound with a compound of formula VI 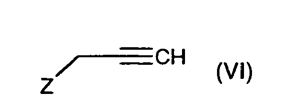 wherein Z signifies phenylsulphonyl, tosyl, methylsulphonyl, nosyl, bromophenyl, Cl-, Br- or CICO- , into the compound of formula I.

## Revendications

1. Procédé de préparation de l'ester propynylique de l'acide (R) (+) -2- [4- (5-chloro-3-fluoropyridine-2-yloxy)-phénoxy]-propionique de formule I 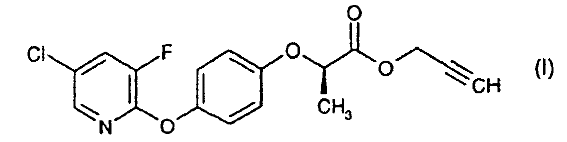 **caractérisé en ce qu'**on convertit un composé de formule II 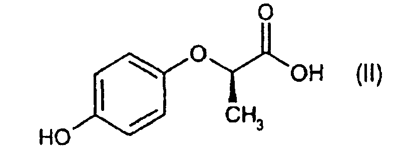 dans un solvant organique inerte sans isolement des produits intermédiaires avec du M₂CO₃, M étant le sodium ou le potassium, en le composé de formule III 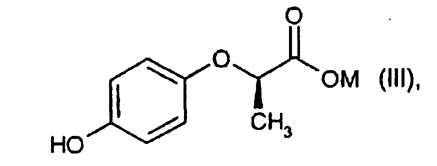 M étant le sodium ou le potassium, on fait réagir ce dernier avec le composé de formule IV 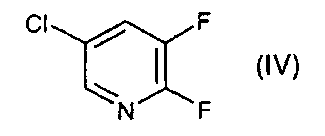 pour obtenir le composé de formule V 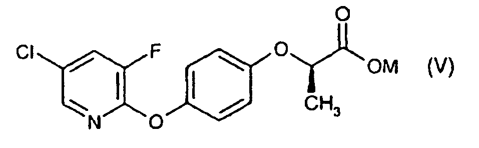 M étant le sodium ou le potassium, et on convertit ce dernier avec un composé de formule VI 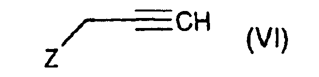 dans laquelle Z est le groupe phénylsulfonyle, tosyle, méthylsulfonyle, nosyle, bromophényle, Cl⁻, Br⁻ ou CLCO⁻, en le composé de formule I.
